# EUROPEAN PATENT SPECIFICATION

(11) **EP 0 527 909 B1**
(45) Date of publication and mention of the grant of the patent: **24.11.1993**
(21) Application number: 91909858.2
(22) Date of filing: 10.05.1991
(51) Int. Cl.: A61B 10/00

(54) **INSTRUMENT FOR ENDO- AND ECTOCERVICAL SAMPLING**
INSTRUMENT ZUR ENDO- UND EKTOCERVIKALEN PROBENNAHME
INSTRUMENT D'ECHANTILLONNAGE ENDO- ET ECTOCERVICAL

(30) Priority: 10.05.1990 SE 9001679
(43) Date of publication of application: 24.02.1993
(73) Proprietor: MEDSCAND AB, S-20074 Malmö (SE)
(72) Inventor: CLAREN, Jan, S-222 47 Lund (SE)
(74) Representative: Ström, Tore
(86) International application number: SE9100332
(87) International publication number: WO9116855

(56) References cited:
- WO-A-89/10724
- US-A- 4 754 764
- US-A- 4 759 376
- US-A- 4 762 133

## Description

The invention relates to an instrument for endo- and ectocervical sampling and has especially been created for cervical cytological sampling in connection with the so-called Pap-test, which is used for establishing the presence of cancer or the preliminary stage of cancer in cervix uteri, but the instrument is also useful for gathering cells, epithelial fragments and secretion from cervix uteri or for other kinds of tests and examinations.

In addition to brushes, also spatulas and cotton swabs are used for cervical sampling but the brush has proven to be far superior to other sampling instruments, especially the embodiment shown and described in US-A-4,759,376, which is marketed under the registered trademark CYTOBRUSH, since by using such a brush cells can be gathered in abundant amounts which are then smeared onto a glass slide while maintaining the cells in an intact condition.

When cytological cervical sampling for the Pap-test is concerned it is necessary, in order to make as confident as possible a diagnosis by guidance of the test, not only to take an endocervical as by using the brush shown in the above cited US patent, but also an ectocervical sample, since cancer or precancer stages in cervix uteri often appear initially in the transformation zone between the area with disc-shaped cells and the area with rod-shaped cells in cervic uteri. It is desired to sample both areas by one and the same instrument and in one and the same operation. Brushes which allow for this have also been proposed.

US-A-4,762,133 thus shows and describes three different embodiments of this type of instrument. In a first embodiment a brush projecting axially in line with the shaft, which narrows towards its free end and is intended for endocervical sampling, is connected with the shaft by a V-shaped wire section projecting laterally from the shaft, one limb thereof forming a second brush with radial bristles and joining the first mentioned axial brush at an angle of about 85°. Said second brush then is intended for the ectocervical sampling. Thus, the instrument is unsymmetrical, which makes it difficult to handle when rotated during sampling for exfoliating the cells, and it is difficult to smear the sample taken ectocervically. Furthermore, the instrument is considerably more complicated in manufacture than the simple axial brush, which is reflected in higher production costs, something that cannot be disregarded concerning such a one-way article as referred to here.

A complicated manufacturing is also characteristic for a second embodiment in US-A-4,762,133, wherein the conventional instrument has an axially extending brush at one end of a shaft which is slid into a tube-shaped second shaft having a circular disc with bristles on one side thereof at one end of said tubular shaft, the axial brush projecting from the center of the bristled area of the disc. Also with this embodiment it is difficult to smear the ectocervical sample that has been collected on the bristled area of the circular disc.

In a third embodiment the conventional instrument with an axially projecting brush at one end of a shaft is combined with a similar instrument with the brush angled radially from the shaft, both shafts extending in parallel and being disconnectibly joined in order that the brushes can be separated before smearing onto the glass slide. There exists a risk of splashing around the obtained sample when disconnecting the shafts, and also this embodiment is unsymmetrical and difficult to rotate while sampling, substantially due to the fact that the shaft is not round.

US-A-4,700,713 shows an instrument which can be used for cervical sampling and which has only axially, flexible bristles at one end of a shaft, said instrument, including the bristles, being injection moulded as a single piece of plastic material. The central bristles project beyond the bristles located at both sides thereof. The central bristles are applied against the cervix while the others are forced to contact the ectocervical area with considerable deformation thereof. At the insertion of the instrument these central bristles can easily be bent aside, these bristles lacking the ability to center the instrument as a consequence thereof, such that the instrument will be incorrectly positioned when sampling. In this connection it should be taken into consideration particularly that portio vaginalis cervicis uteri is soft and has a heterogenous shape, implying a possible variation in the anatomy from one individual to another. This earlier known instrument thus does not provide the stability that is required in order to correctly position the instrument for sampling. A great amount of cells get stuck between the bristles and remain there when smearing the sample, which is an additional disadvantage. Furthermore, the instrument cannot be rotated while smearing but must be moved linearily across the glass slide on which the smearing takes place, cells and secretion from the endocervical area being smeared as a small band between broader bands of cells and secretion from the ectocervical area. Besides, this small band is diffuse, and the analysis of the sample obtained thus becomes difficult to accomplish and involves great uncertainty concerning the results.

In order to reduce the manufacturing costs without impairing the function of the instrument there has been proposed according to the international publication WO 89/10724 an instrument for cervical sampling having a brush consisting of a velour finished surface with fibres which are attached to the surface and stand on end projecting from the surface. In one embodiment of this prior art instrument a velour finished surface is provided on a conical end section of a shaft for insertion into cervix uteri, and two flexible, obliquely backwards protruding flaps having a velour finished surface, connect to the rear end of the velour finished conical end section. Also with this instrument sampling can take place in both the endocervical area and the ectocervical area but it may be difficult to collect enough cells from the ectocervical area since the flaps are easily bent aside when pressure is exerted on the instrument and you do not really know where the flaps are located during the actual sampling.

The purpose of the present invention is to produce an instrument for endo- and ectocervical sampling having velour finished surfaces in order to keep the manufacturing costs considerably lower than if bristles are fastened to a twisted metal wire, and being constructed such that it can be placed in its correct position for sampling irrespective of existing anatomical differences of those individuals who are subjected to sampling and, moreover, allows for a reliable gathering of cells and secretion from the endocervical area and the ectocervical area as well as the transformation zone therebetween with subsequent smearing of cells and secretion onto a glass slide in two close to each other adjoining broad parallel bands permitting a safe analysis of the sample taken.

The instrument according to the invention comprises in a known manner a shaft with an axially oriented brush section at one end of the shaft and with a pair elastically flexible brush sections connecting to the rear end of the axial section on opposite sides of the shaft the brush sections being constructed as velour finished areas with fibres attached to these areas and standing on end projecting therefrom as disclosed in WO 89/10724.

In order to achieve this purpose, the instrument according to the invention has obtained the characterizing features of claim 1.

In order to further explain the invention two embodiments thereof will be described below reference being made to the accompanying drawings, in which
FIG 1 is a plan view of the instrument according to the invention in one embodiment thereof,
FIG 2 is a fragmentary side view of the instrument in FIG 1,
FIG 3 illustrates the use of the instrument for sampling,
FIG 4 illustrates smearing of the sample obtained from the instrument onto a glass slide,
FIG 5 is a plan view of the instrument according to the second embodiment of the invention,
FIG 6 is a fragmentary side view of the instrument in FIG 5,
FIG 7 illustrates the use of the instrument for sampling, and
FIG 8 illustrates smearing of the sample obtained from the instrument onto a glass slide.

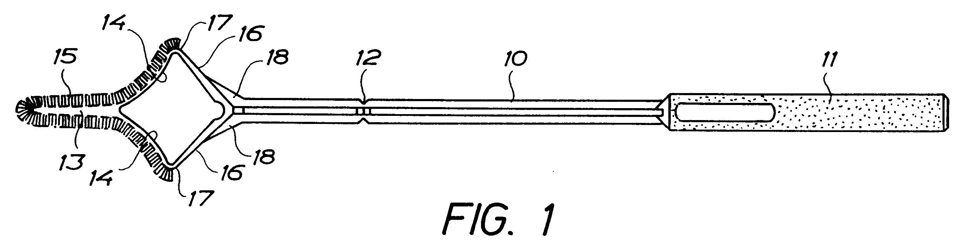

The instrument according to FIG 1-4 for endo- and ectocervical sampling comprises an injection moulded shaft 10 of thermoplastics. In order to have the required strength and resilience the shaft is constructed with a cruciformed cross section and it has at one end thereof a handle section 11 and near its other end a fractured impression 12. In the last-mentioned end a sampling brush is connected, but this brush can also be integrated with the shaft 10. The brush is injection moulded of thermo-plastics and comprises a slightly conical end section 13 to the rear end of which two flap-shaped sections 14 are connected on opposite sides of the section 13. The sections 14 are elastically flexible. The section 13 is velour finished on its curved surface as well as on the end surface at the end of the instrument, and the sections 14 are velour finished on their outside surface in such a way that the sections 13 and 14 form a continuous brush surface 15. Velour finishing can be accomplished as described in WO 89/10724 or in other ways. A pair of arms 16 are made integral with with the sections 14 and with the shaft or a pin, which is fixed to the shaft, and these arms form together with the sections 14 a substantially regular tetragon, in the present case a rhomb and almost a square, the diagonals of which cross each other at substantially right angles within the tetragon. The hinges 17 between the arms 16 and the sections 14 preferably consist of thinner portions of the plastic material, having such a rigidity that the instrument normally exhibits the configuration shown in FIG 1. The sections 14 are slightly curved to have a concave shape on the brush outside. The arms 16 are slightly wider than the sections 14 which taper from the width of the arms towards the section 13. Stiffening flanges 18 are arranged between the shaft and the arms in order to stiffen the arms close to the shaft, the arms otherwise being elastically flexible. By this construction of the arms the instrument achieves the stability required in order to smear a sample therefrom.

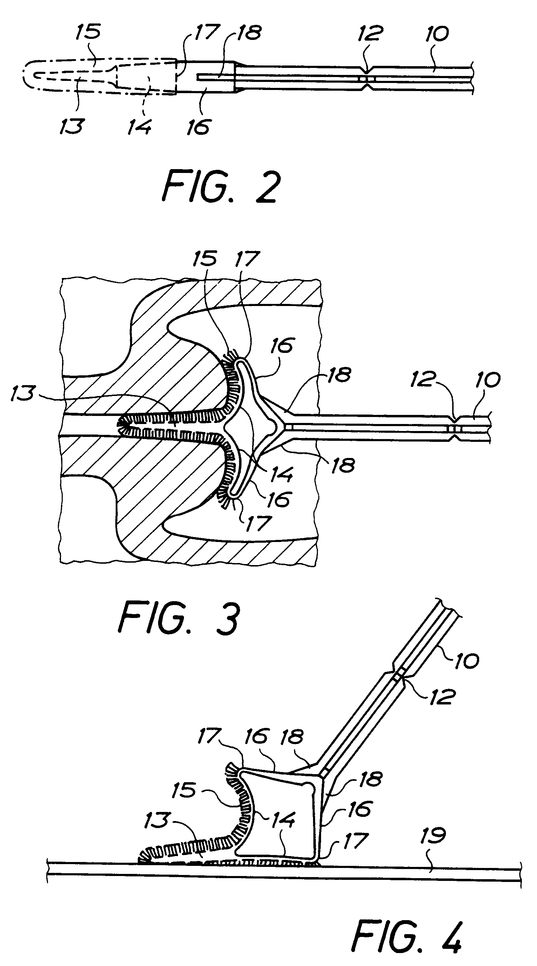

FIG 3 disclosed the use of the instrument described. In FIG 3 it is shown with the end section 13 inserted into cervix uteri and with the flexible sections 14 applied against the ectocervical area, pressure being exerted on the shaft 10 to press the sections 14 against this area, which means that the square configuration formed by the sections 14 and the arms 16 has been deformed. When the instrument is now rotated in the position shown in FIG 3 the brush formed by the velour finished sections will scrape cells and secretion from the endocervical area and the ectocervical area and also from the transformation zone between these areas, which is important to sample since an incipient cancer first of all is revealed by cell changes in this transformation zone. The velour finished surface causes minimal pain during sampling and is very effective with respect to gathering cells and secretion.

When the sampling has taken place and the instrument has been removed from the patient a smearing onto a glass slide 19 takes place in the manner shown in FIG 4, one side of the end section 13 and one of the flexible sections 14 being smeared initially against the glass slide and then the other side and the other section. By this smearing technique the section 13 and one of the sections 14 are located mainly planely in level against the glass slide so that by smearing onto the glass slide two bands are obtained where the transformation zone easily can be localized between both the areas representing the endocervical area and the ectocervical area, respectively. The analysis of the sample on the glass slide is thus considerably facilitated, implying a reliable diagnosis.

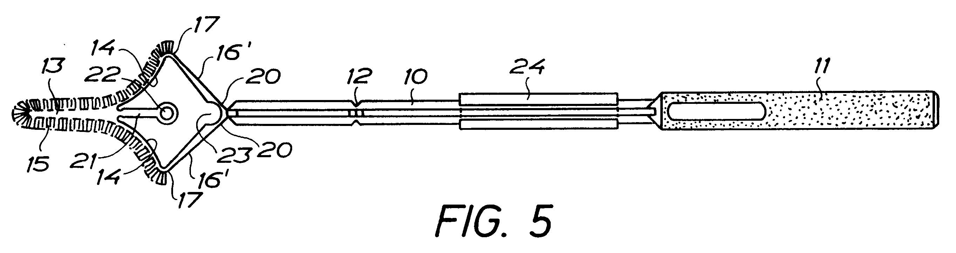

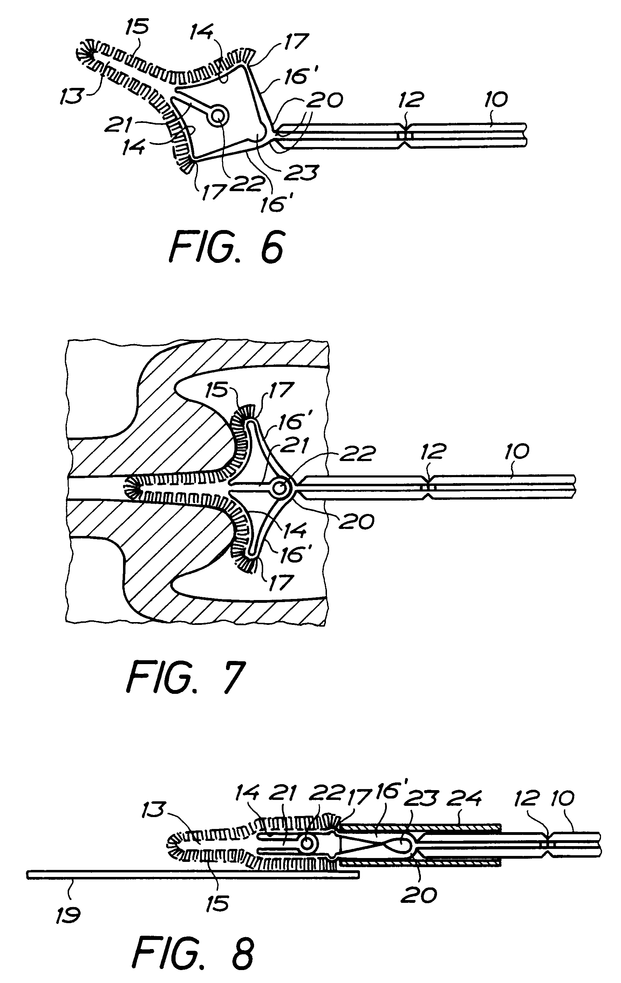

In the second embodiment according to the invention, FIGS 5-8, the arms 16 in the described embodiment are replaced by links 16', which are connected with the shaft 10 by hinges, which like the hinges 17 suitably are composed of thinner portions of the plastic material. The hinges 20 have such a rigidity that the substantially right angled square at the end section 13 can be tilted laterally, FIG 6, said square at the same time having such a rigidity that the end section 13 without difficulty can be inserted into cervix uteri with the square in its tilted position. A spacer 21, the free end of which forms an eye 22, is connected to the base of the conical end section 13 between the sections 14. In the junction between the links 16' a cavity 23 is arranged which can receive the eye 22. On the shaft 10 a slide 24 is provided which is displaceable along the shaft, and this slide can be pushed over the links 16' against the hinges in order to move the links towards each other.

FIG 7 illustrates the use of the second embodiment of the described instrument for sampling. It is shown in FIG 7 with the end section 13 inserted into cervix uteri and with the flexible sections 14 applied against the ectocervical area. When pressure is exerted on the shaft 10 for pressing the sections 14 against the ectocervical area by the pressure being transferred from the shaft to the outer parts of the sections 14 via the links 16', the eye 22 on the spacer 21 will engage one of the links 16' and may be received by the cavity 23, the spacer in said latter case engaging the shaft 10 and thus stabilizing the end section 13.

When the instrument has been rotated for sampling and is withdrawn from the patient, smearing of the gathered sample onto a glass slide is done in the way shown in FIG 8. In this case the slide 24 is displaced over the links 16' against the hinges 17, positioning the continuous brush surface 15 almost linearly between the tip of the end section 13 over the sections 14 to the hinges 17. Then, the sections 14 are separated by the eye 22 on the spacer 21. Smearing onto the glass surface of the two sides of the instrument can then be accomplished as described above.

The described embodiments can of course be modified. As mentioned above, the instrument as a whole can be constructed in one piece only, but it is also possible to manufacture the part formed by the sections 14, 15 and 16, or the sections 14, 15, 16' and 21, respectively, as a separate part, which is fitted to the shaft, and then the possibility obviously exists to construct the shaft by extrusion with the handle section 11 having the same cross section as the rest of the shaft. The tetragon formed by the sections 14 and 16, or the sections 14 and 16', respectively, is not necessarily a rhomb or a square as shown here; the arms 16 and 16', respectively, can have a greater length than the sections 14.

The spacer 21 of the embodiment according to FIGS 5-8 can also in a corresponding way be provided in the embodiment according to FIGS 1-4. The section 13 is shown slightly conical but can also have a substantially cylindrical shape.

The fibres that form the brush 15 can be dimensioned as disclosed in WO 89/10724, i.e. the fibres in a typical case have a length of the order of 2 mm and a diameter of the order of 0.12 mm to cause the smallest possible tendency of bleeding during sampling. A suitable plastics for manufacturing of the instrument is polypropylene but other plastic materials also suitable for the instrument according to the invention may be suggested. By the arrangement of the fracture impression 12 the end section of the instrument, i.e. the part containing the sample obtained in the brush section 15, can be broken off when put into a test tube used for treating or transporting the sample obtained.

## Claims

1. Instrument for endo- and ectocervical sampling comprising a shaft (10) with an axially oriented brush section (13) of one end of said shaft and with a pair elastically flexible brush sections (14) connecting to the rear end of the axial section on opposite sides of said shaft the brush sections being constructed as velour finished areas with fibres attached to these areas standing on end projecting therefrom
**characterized** in that said flexible brush sections (14) are connected with said shaft (10) by means of two arms (16; 16') hingedly connected at one end thereof with the outer ends of said flexible brush sections, said arms at the other end thereof being connected with the shaft and together with the flexible brush sections forming the configuration of a substantially regular tetragon, the diagonals of which cross each other at substantially right angles within said tetragon, said axial section, said flexibly brush sections and said arms being formed integrally of a plastic material, the hinges (17) being arranged with such a rigidity that the instrument normally exhibits said configuration.

2. Instrument as in claim 1,
**characterized** in that said arms (16) are substantially rigidly connected with the shaft (10) and are elastically flexible.

3. Instrument as in claim 2,
**characterized** in that the arms (16) are stiffened in an area adjacent to said shaft (10).

4. Instrument as in claim 3,
**characterized** in that said stiffness is accomplished by means of corner flanges (18) between the shaft (10) and the arms (16).

5. Instrument as in claim 1,
**characterized** in that said arms are arranged as links (16').

6. Instrument as in claim 5,
**characterized** in that a slide (24) is arranged on said shaft (10) and is displaceable over said links (16') against the hinges (17).

7. Instrument as in any of claims 1-6,
**characterized** in that a spacer (21) projecting towards the shaft is connected to the base of said axial brush section (13) between said flexible brush sections (14) the free end of said spacer being engageable with the end of the shaft (10) and/or said links (16').

8. Instrument as in claim 7,
**characterized** in that a cavity (23) is provided at the end of said shaft (10) for receiving the end (22) of the spacer (21).

9. Instrument as in any of claims 1-8,
**characterized** in that the hinges (17, 20) are arranged as thinner portions of the plastic material.

10. Instrument as in any of claims 1-9,
**characterized** in that said tetragon has the shape of a square or a rhomb.

11. Instrument as in any of claims 1-10,
**characterized** in that said flexible brush sections (14) are curved in order to exhibit an outside concave surface.

12. Instrument as in any of claims 1-11,
**characterized** in that said flexible brush sections (14) taper from said arms (16; 16') towards said axial brush section (13).

## Patentansprüche

1. Instrument zur endo- und ektozervikalen Probennahmen, daß aufweist, einen Stiel (10) mit einem axial orientierten Bereich (13) anein em Ende des Stiels und mit einem Paar elastisch flexibler Bürstenbereiche (14), die am rückwärtigen Ende des axialen Bereichs an gegenüber liegenden Seiten des Stiels angeordnet sind, wobei die Bürstenbereiche als Bereiche mit Velouroberfläche ausgebildet sind und Fasern aufweisen. die zum Ende hin vorspringen und von innen vorstehen, dadurch gekennzeichnet, daß die fle xiblen Bürstenbereiche (14) mit dem Stiel (10) über zwei Arme (16; 16') an einem Ende mit den äußeren Enden der genannten flexiblen Bürstenbereiche gelenkverbunden sind, daß diese Arme an ihrem anderen Ende mit dem Stiel verbunden sind und daß sie zusammen mit den flexiblen Bürstenbereichen die Konfiguration eines im wesentlichen regelmäßigen Vierecks bilden, dessen Diagonalen sich im wesentlichen unter rechten Winkeln innerhalb des Vierecks schneiden, daß der axiale Bereich, die flexiblen Bürstenbereiche und die Arme einstückig aus Kunststoff gefertigt sind, und daß die Gelenkbereiche (17) mit einer derartigen Steifigkeit ausgestattet sind, daß das Instrument normalerweise die angegebene Konfiguration einnimmt.

2. Instrument nach Anspruch 1, dadurch gekennzeichnet, daß die Arme (16) im wesentlichen starr mit dem Stiel (10) verbunden und elastisch flexibel sind.

3. Instrument nach Anspruch 2, dadurch gekennzeichnet, daß die Arme (16) in ihrem Bereich in Nähe des Stiels (10) ausgesteift sind.

4. Instrument nach Anspruch 3, dadurch gekennzeichnet, daß die Steifigkeit erreicht wird durch zwei Eckflansche (18) zwischen dem Stiel (10) und den Armen (16).

5. Instrument nach Anspruch 1, dadurch gekennzeichnet, daß die Arme als Gelenkarme (16') ausgeführt sind.

6. Instrument nach Anspruch 5, dadurch gekennzeichnet, daß ein Schlitten (24) am Stiel (10) vorgesehen ist, der über die Gelenkarme (16') gegen die Gelenkbereiche (17) verschiebbar ist.

7. Instrument nach einem der Ansprüche 1 - 6, dadurch gekennzeichnet, daß ein Abstandsteil (21) zum Stiel hin vorspringt und mit der Basis des axialen Burstenbereichs (13) zwischen den flexiblen Bürstenbereichen (14) verbunden ist, wobei das freie Ende dieses Abstandsteils mit dem Endbereich des Stiels (10) undloder den genannten Gelenkarmen (16') in Eingriff kommen kann.

8. Instrument nach Anspruch 7, dadurch gekennzeichnet, daß eine Ausnehmung (23) am Ende des Schafts (10) vorgesehen ist, um das Ende (22) des Abstandsteils (21) aufzunehmen.

9. Instrument nach einem der Ansprüche 1 - 8, dadurch gekennzeichnet, daß die Gelenkbereiche (17, 20) als verdünnte Querschnittsbereiche des Kunststoffmaterials ausgebildet sind.

10. Instrument nach einem der Ansprüche 1 - 9, dadurch gekennzeichnet, daß das Viereck die Form eines Quadrats oder eines Rhombus hat.

11. Instrument nach einem der Ansprüche 1 - 10, dadurch gekennzeichnet, daß die flexiblen Bürstenbereiche (14) gekrümmt sind, sodaß sie außenseitig eine konkave Oberfläche ausbilden.

12. Instrument nach einem der Ansprüche 1 - 11, dadurch gekennzeichnet, daß die flexiblen Bürstenbereiche (14) ausgehend von den Armen (16, 16') zu dem axialen Bürstenbereich (13) hin schmaler werden.

## Revendications

1. Instrument de prélévement endocervical et ectocervical comprenant un manche (10) avec une section de brosse (13), orientée axialement, à une extrémité du manche, et avec une paire de sections de brosse (14) flexibles élastiquement, reliées à l'extrémité arrière de la section axiale, des deux côtés opposés du manche, les sections de brosse étant réalisées sous la forme de zones à fini velouté avec des fibres attachées à ces zones et se dressant sur une extrémité faisant saillie à partir de celles-ci, caractérisé en ce que les sections de brosse flexibles (14) sont reliées au manche (10) par l'intermédiaire de deux bras (16,16') articulés, à l'une de leurs extrémités, avec les extrémités externes des sections de brosse flexibles, ces bras étant reliés, à l'endroit de leurs autres extrémités, au manche et formant, conjointement avec les sections de brosse flexibles, un quadrilatère sensiblement régulier dont les diagonales se recoupent l'une ou l'autre suivant des angles sensiblement droits à l'intérieur du quadrilatère, la section axiale, les sections de brosse flexibles et les bras étant formés intégralement d'une matière plastique, les articulations (17) étant agencées de maniére à avoir une rigidité telle que l'instrument présente normalement la configuration précitée.

2. Instrument suivant la revendication 1 caractérisé en ce que les bras (16) sont reliés d'une manière sensiblement rigide au manche (10) et ils sont flexibles élastiquement.

3. Instrument suivant la revendication 2 caractérisé en ce que les bras (16) sont raidis dans une zone voisine du manche (10).

4. Instrument suivant la revendication 3 caractérisé en ce que le raidissement est réalisé au moyen de goussets (18) entre le manche (10) et les bras (16).

5. Instrument suivant la revendication 1 caractérisé en ce que les bras sont agencés en tant que bras articulés (16').

6. Instrument suivant la revendication 5 caractérisé en ce qu'un coulisseau (24) est monté sur le manche (10) et il peut être déplacé par-dessus les bras articulés (16'), contre les articulations (17).

7. Instrument suivant l'une quelconque des revendications 1 à 6 caractérisé en ce qu'une entretoise (21), s'étendant en direction du manche, est reliée à la base de la section de brosse axiale (13), entre les sections de brosse flexibles (14), l'extrémité libre de cette entretoise pouvant venir en contact avec l'extrémité du manche (10) et/ou avec les bras articulés (16').

8. Instrument suivant la revendication 7 caractérisé en ce qu'une cavité (23) est prévue à l'endroit de l'extrémité du manche (10) pour recevoir l'extrémité (22) de l'entretoise (21).

9. Instrument suivant l'une quelconque des revendications 1 à 8 caractérisé en ce que les articulations (17,20) sont réalisées sous la forme de parties plus minces de la matière plastique.

10. Instrument suivant l'une quelconque des revendications 1 à 9 caractérisé en ce que le quadrilatère a la forme d'un carré ou d'un losange.

11. Instrument suivant l'une quelconque des revendications 1 à 10 caractérisé en ce que les sections de brosse flexibles (14) sont incurvées de manière à présenter une surface externe concave.

12. Instrument suivant l'une quelconque des revendications 1 à 12 caractérisé en ce que les sections de brosse flexibles (14) ont une forme convergente à partir des bras (16,16') et en direction de la section de brosse axiale (13).
